# FASCICULE DE BREVET EUROPEEN

(11) **EP 4 200 590 B1**
(45) Date de publication et mention de la délivrance du brevet: **18.03.2026**
(21) Numéro de dépôt: 21763064.9
(22) Date de dépôt: 17.08.2021
(51) Int. Cl.: A61K 8/73, A61Q 19/00, G01N 11/00, A61K 8/04, G01N 11/16

(54) **PROCEDE D'EVALUATION DES CARACTERISTIQUES RHEOLOGIQUES D'UN GEL**
VERFAHREN ZUR AUSWERTUNG RHEOLOGISCHER EIGENSCHAFTEN EINES GELS
METHOD FOR EVALUATING RHEOLOGICAL PROPERTIES OF A GEL

(30) Priorité: 20.08.2020 FR 2008590
(43) Date de publication de la demande: 28.06.2023
(73) Titulaire: Laboratoires Vivacy, 75116 Paris (FR)
(72) Inventeur: TRANCHEPAIN, Frédéric, 74350 VILLY LE BOUVERET (FR); BRUNEL, Florence, 74100 ANNEMASSE (FR); BIGAND, Virginie, 74370 PRINGY (FR); VINCENT, Sandra, 74160 PRESILLY (FR)
(74) Mandataire: Tripoz, Inès
(86) Numéro de dépôt international: PCT/EP2021/072864
(87) Numéro de publication internationale: WO 2022/038156

(56) Documents cités:
- WO-A1-2016/150974
- WO-A1-2018/104426
- WO-A1-2020/212615

## Description

L'invention concerne un procédé d'évaluation des caractéristiques rhéologiques d'un gel, notamment d'un hydrogel à base d'acide hyaluronique, permettant notamment la différenciation de produits comparables en termes d'utilisation ou de préconisations d'usage.

De tels gels, peuvent être des hydrogels utilisés comme gels injectables en chirurgie esthétique, des gels utilisables dans le domaine des traitements articulaires ou des gels utilisés dans des applications médicales comme la cicatrisation voir en tant que dispositifs de relargage et diffusion de principes actifs pharmaceutiques.
Dans le cadre de la présente demande, un certain nombre de définitions sont ci-après rappelées.

Le gel, selon l'UICPA (Union internationale de chimie pure et appliquée) est décrit comme un réseau colloïdal non fluide ou un réseau polymère qui est étendu dans tout son volume par un fluide.

Parmi les gels on distingue les hydrogels définis comme des gels polymériques constitués d'un réseau tridimensionnel constitué d'au moins un polymère susceptible d'absorber une grande quantité d'eau ou de solution aqueuse et qui présentent des propriétés rhéologiques particulières notamment en termes de viscosité et de viscoélasticité.

Dans un gel ou un hydrogel, ledit réseau peut être constitué par des réticulations chimiques par création de liaisons entre les chaines polymériques, ces liaisons pouvant être des liaisons covalentes. Ledit réseau peut également être obtenu par interactions physiques transitoires par exemple des liaisons ioniques, hydrophobes ou hydrogènes.

Les caractéristiques rhéologiques ou les propriétés rhéologiques sont les caractéristiques physiques qui permettent l'étude de la déformation et de l'écoulement de la matière sous l'application d'une contrainte.

S'agissant des caractéristiques rhéologiques déterminées pour des hydrogels, on peut citer le module élastique G' et le module visqueux G". Ces données sont obtenues par soumission d'un échantillon à un balayage en fréquence à déformation fixe de faible amplitude (dans le domaine linéaire) qui est une méthode bien connue de l'homme du métier.

Or, ces balayages en fréquences sont assimilables à des photographies des réseaux prises en statique et ne sont pas suffisants pour permettre de prédire et/ou déterminer le comportement dynamique des gels sous différentes contraintes ou déformations *in-vivo* et pour différencier et comparer des produits.

Cependant, l'évaluation des caractéristiques rhéologiques d'un gel est indispensable lors de développement de produits car elle permet de limiter le nombre de tests au laboratoire pour évaluer les performances des gels et faire des choix sans avoir la nécessité de recourir systématiquement à des tests *in vivo.*

De plus, l'évaluation des caractéristiques rhéologiques d'un gel permet de faciliter le développement de nouveaux types de gels et de travailler les propriétés du gel pour obtenir le résultat souhaité en fonction des différentes applications.

Dans l'art antérieur, des méthodes d'évaluation des caractéristiques rhéologiques de gels injectables complémentaires à la détermination des modules élastique G' et visqueux G" ont été décrites.

Par exemple, dans le brevet EP3274685B1 au nom de Teoxane, il est divulgué un procédé d'évaluation des caractéristiques rhéologiques d'un gel injectable. Ce procédé porte sur une méthode de mesure de l'aire sous la courbe de G' entre deux bornes de mesures a priori déterminées arbitrairement, afin de donner une indication sur la résistance mécanique du gel, c'est-à-dire sur la capacité que le gel a de maintenir sa structure sur un spectre étendu de valeurs. Ce score d'intégrale est une grandeur physique homogène à une énergie. Cependant, pour différencier deux gels qui auraient un « score d'intégrale » similaire, ledit score étant dépendant du choix des bornes d'intégration et de la méthode d'intégration, un autre paramètre doit être mesuré en complément. Il s'agit du « creep measurement » qui est une grandeur associée à la déformation élastique et visqueuse immédiate ou dans le temps. D'après cette même demande, cette grandeur est reliée au « slope of the creep » qui est calculé comme étant le ratio entre la contrainte imposée et la viscosité du gel et est homogène à s⁻¹.

Comme illustré à la Figure 15, la présente invention se distingue de la méthode divulguée dans la demande de brevet WO2016/150974 ou dans le brevet EP3274685B1. La présente invention se distingue par l'étude de paramètres physiques différents de ceux établis dans la demande de brevet WO2016/150974 ou dans le brevet EP3274685B1 afin de différencier et/ou comparer deux gels distincts. De plus, la présente invention permet de mesurer et d'étudier le comportement de l'hydrogel soumit à des contraintes et/ou des déformations dans le domaine plastique de manière directe par la détermination de la plage de résistance, ladite plage de résistance étant définie par la différence ζ_{C} -ζ_{P}, soit l'étendue du domaine plastique en contrainte et de la plage de malléabilité, ladite plage de malléabilité étant définie par la différence γ_{C}-γ_{P} , soit l'étendue du domaine plastique en déformation.

Dans la demande de brevet WO2018/104426 au nom de Nestlé Skin Health est divulgué un procédé d'évaluation des caractéristiques rhéologiques d'un gel injectable. Ce procédé s'appuie sur la mesure d'un paramètre appelé la flexibilité. Ce paramètre physique est évalué par la mesure de la contrainte au point de croisement du balayage d'amplitude entre le module élastique G' et le module visqueux G".

Ainsi, ces méthodes permettent de compléter les mesures classiques d'élasticité/rigidité et viscosité mais elles ne permettent pas de mesurer et d'étudier le comportement de l'hydrogel soumis à des contraintes et/ou des déformations dans le domaine plastique.

La présente invention permet de mesurer et d'étudier le comportement de l'hydrogel soumit à des contraintes et/ou des déformations dans le domaine plastique et consiste en un procédé d'évaluation des caractéristiques rhéologiques d'au moins un gel, consistant en la détermination de la plage de résistance, ladite plage de résistance étant définie par la différence ζ_{C}-ζ_{P}, soit l'étendue du domaine plastique en contrainte et de la plage de malléabilité, ladite plage de malléabilité étant définie par la différence γ_{C}-γ_{P} , soit l'étendue du domaine plastique en déformation.

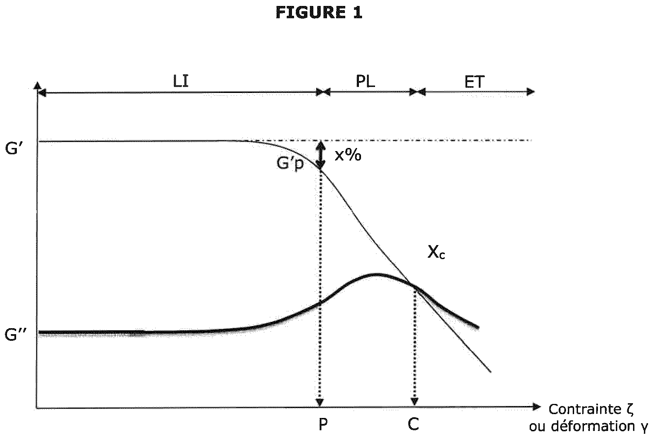

La Figure 1 est une représentation schématique de l'évolutions des modules visqueux et élastique d'un échantillon de gel soumit à un balayage en déformation et/ou en contrainte et des domaines de linéarité noté LI, plastique PL et d'étalement ET.

Le module élastique G', est une grandeur intrinsèque d'un matériau, gel en l'occurrence, définie par le rapport de la contrainte à la déformation élastique . Le module élastique est exprimé en Pascal.

Le module visqueux G" ou module de dissipation est une grandeur physique qui caractérise le comportement visqueux lié à la capacité à dissiper la contrainte élastique. Le module visqueux est exprimé en Pascal.

Le rapport du module visqueux sur le module élastique est défini comme le facteur de perte mécanique, noté tan δ.

Le balayage en déformation permet l'étude dynamique de G' et G" en fonction de la déformation pour une fréquence d'oscillation fixée.

Le balayage en contrainte permet l'étude dynamique de G' et G" en fonction de la contrainte pour une fréquence fixée.

Comme illustré à la Figure 1, le domaine de linéarité est le domaine dans lequel le gel n'est pas affecté par la contrainte, il est considéré au repos, c'est-à-dire que G' est considéré comme constant. Ce domaine, noté LI sur le schéma, correspond au domaine délimité par la valeur d'origine de l'axe des abscisses et la valeur P sur l'axe des abscisses.

Le domaine d'étalement est le domaine dans lequel le gel présente une déstructuration complète du réseau. Comme illustré à la Figure 1, ce domaine, noté ET sur le schéma, correspond au domaine au-delà de la valeur C sur l'axe des abscisses.

Le domaine plastique est le domaine dans lequel le gel est malléable et présente des réarrangements au niveau des liaisons physiques rompues localement.

Comme illustré à la Figure 1, les bornes de début et de fin du domaine plastique, noté PL sur le schéma, sont représentées respectivement par les points P et C sur l'axe des abscisses et ce domaine.

Le point P est déterminé arbitrairement lorsque la valeur du module élastique G' a diminué de x%.

Comme illustré à la Figure 1, sa valeur est déterminée sur l'axe des abscisses lorsque G' est égal à G'p.

Comme illustré à la Figure 1, le point C représente la valeur des abscisses au point d'intersection des courbes de G' et G" à une fréquence d'oscillation fixe. Ce point d'intersection est noté Xc sur la Figure 1.

Lors du balayage en contrainte, les points P et C représentés sur la Figure 1 désignent respectivement les variables ζ_{C} et ζ_{P}, exprimées en Pascal.

Lors du balayage en déformation, les points P et C représentés sur la Figure 1 désignent respectivement les variables γ_{P} et γ_{C}. Ces variables sont exprimées en % de déformation et sont adimensionnelles.

La plage de résistance peut être définie comme l'intervalle de forces qui peuvent être appliquées au gel pour le déformer sans le déstructurer définitivement. Au-delà de cette plage, le gel a perdu son intégrité. Elle sera déterminée par l'étendue du domaine plastique en contrainte, à savoir par la valeur de la différence entre ζ_{C} et ζ_{P}.

Cette plage de résistance permet de quantifier la résistance du gel aux contraintes et forces auxquelles il est soumis *in-vivo,* par exemple dans des lèvres en mouvement ou dans une articulation. Elle va permettre d'apprécier la capacité du gel à casser et à se fractionner sous une pression externe.

La plage de malléabilité peut être définie comme la capacité du gel à se déformer et à être modelé tout en conservant ses caractéristiques rhéologiques initiales. Elle sera déterminée par l'étendue du domaine plastique en déformation à savoir par la valeur de la différence entre γ_{C} et γ_{P}.

La plage de malléabilité va permettre d'apprécier l'adaptation et la possibilité de réponse à la dynamique et aux mouvements des tissus environnants. Elle permettra également de prédire l'aptitude du produit à être « modelé » en fonction du besoin et selon l'indication.

La présente invention est un procédé d'évaluation des caractéristiques rhéologiques d'au moins un gel, consistant en la détermination de l'étendue du domaine plastique en contrainte ζ_{C}-ζ_{P} et en déformation γ_{C}-γ_{P}, ladite détermination étant réalisée selon les étapes de :
- soumission d'au moins un échantillon d'au moins un gel à des contraintes mécaniques oscillatoires à une fréquence fixée,
- détermination et tracé des courbes du module élastique G' et du module visqueux G" en fonction de la déformation et de la contrainte,
- détermination de ζ_{C} et γ_{C} au point d'intersection (Xc) des courbes G' et G" en contrainte et en déformation,
- détermination de ζ_{P} et γ_{P} par fixation d'une valeur arbitraire de G' (G'p) que l'on définira comme la valeur d'entrée dans le domaine plastique et,
- calcul de ζ_{C} -ζ_{P} et de γ_{C}-γ_{P}.

Dans un mode de réalisation, les contraintes mécaniques oscillatoires sont mises en œuvre par un balayage en amplitude.

Dans un mode de réalisation, le balayage en amplitude désigne à la fois le balayage en déformation et le balayage en contrainte.

Dans un mode de réalisation, la valeur arbitraire de G' est définie comme la valeur pour laquelle le module élastique G' a diminué d'au plus 15% par rapport à la valeur de G' au plateau du domaine linéaire, notée G'p.

Le pourcentage minimum de diminution du module élastique G' est dépendant de la sensibilité des mesures effectuées qui est inférieure ou égale à 5%.

Dans un mode de réalisation, la valeur arbitraire de G' est définie comme la valeur pour laquelle le module élastique G' a diminué de 5 à 15% par rapport à la valeur de G' au plateau du domaine linéaire, notée G'p.

Dans un mode de réalisation, la valeur arbitraire de G' est définie comme la valeur pour laquelle le module élastique G' a diminué de 5 à 10% par rapport à la valeur de G' au plateau du domaine linéaire, notée G'p.

Dans un mode de réalisation, la valeur arbitraire de G', est définie comme la valeur pour laquelle le module élastique G' a diminué de 5% par rapport à la valeur de G' au plateau du domaine linéaire, notée G'p en contrainte et en déformation.

Dans un mode de réalisation, la valeur arbitraire de G' est définie comme la valeur pour laquelle le module élastique G' a diminué de 7,5% par rapport à la valeur de G' au plateau du domaine linéaire, notée G'p en contrainte et en déformation.

Dans un mode de réalisation préférentiel, la valeur arbitraire de G' est définie comme la valeur pour laquelle le module élastique G' a diminué de 10% par rapport à la valeur de G' au plateau du domaine linéaire, notée G'p en contrainte et en déformation.

Dans un mode de réalisation, la fréquence fixée lors du balayage en déformation et/ou en contrainte est comprise entre 0,1 et 10 Hz.

Dans un mode de réalisation, la fréquence fixée lors du balayage en déformation et/ou en contrainte est comprise entre 0,5 et 5 Hz.

Dans un mode de réalisation, la fréquence fixée lors du balayage en déformation et/ou en contrainte est comprise entre 0,1 et 5 Hz.

Dans un mode de réalisation, la fréquence fixée lors du balayage en déformation et/ou en contrainte est comprise entre 0,1 et 3 Hz.

Dans un mode de réalisation, la fréquence fixée lors du balayage en déformation et/ou en contrainte est comprise entre 0,5 et 3 Hz.

Dans un mode de réalisation, la fréquence fixée lors du balayage en déformation et en contrainte est comprise entre 0,7 et 2,5 Hz.

Dans un mode de réalisation, la fréquence fixée lors du balayage en déformation et/ou en contrainte est égale à 0,1 Hz.

Dans un mode de réalisation, la fréquence fixée lors du balayage en déformation et/ou en contrainte est égale à 0,5 Hz.

Dans un mode de réalisation, la fréquence fixée lors du balayage en déformation et/ou en contrainte est égale à 0,7 Hz.

Dans un mode de réalisation, la fréquence fixée lors du balayage en déformation et/ou en contrainte est égale à 1 Hz.

Dans un mode de réalisation, la fréquence fixée lors du balayage en déformation et/ou en contrainte est égale à 2,5 Hz.

Dans un mode de réalisation, la fréquence fixée lors du balayage en déformation et en contrainte est égale à 3 Hz.

Dans un mode de réalisation, la fréquence fixée lors du balayage en déformation et/ou en contrainte est égale à 5 Hz.

Dans un mode de réalisation, l'échantillon dont les propriétés rhéologiques sont étudiées en appliquant le procédé est un gel.

Dans un mode de réalisation ledit gel est choisi dans le groupe comprenant les hydrogels, les gels silicones (par exemple un gel de polydiméthylsiloxane), les gels acryliques (par exemple à partir de particules de polyméthacrylate de méthyle), les gels de polyacrylamide.

Dans un mode de réalisation, le gel dont les propriétés rhéologiques sont étudiées en appliquant le procédé selon l'invention est un hydrogel.

Dans un mode de réalisation, ledit hydrogel est choisi dans le groupe comprenant : les polysaccharides, les polyacrylamides, l'alcool polyvinylique, l'acide polylactique, l'acide polyglycolique, les poly(acides lactique-co-glycolique), les poloxamères, le polyéthylène glycol, le poly(N-isopropylacrylamide), la gélatine et le collagène, seuls ou en mélange

Dans un mode de réalisation, ledit hydrogel est choisi dans le groupe des polysaccharides comprenant : le dextrane, la cellulose, l'amidon et les amidons modifiés, les cyclodextrines et ses dérivés, les dérivés de cellulose (notamment l'hydroxypropylcellulose, l'hydroxypropylméthylcellulose, l'éthylméthyle cellulose, la carboxyméthylcellulose), l'acide alginique, le xanthane, le carraghénane, le chitosane, l'agarose, la pectine, les glycosaminoglycanes (GAG) et leur sels biologiquement acceptables, seuls ou en mélange.

Dans un mode de réalisation, ledit polysaccharide est choisi dans le groupe des glycosaminoglycanes (GAG), comme par exemple la chondroïtine, le kératane, l'héparine, l'héparosan ou encore l'acide hyaluronique, et leur sels biologiquement acceptables, seuls ou en mélange.

Dans un mode de réalisation, ledit polysaccharide est l'acide hyaluronique, ou l'un de ses sels biologiquement acceptables, seuls ou en mélange.

Dans un mode de réalisation, ledit polysaccharide est de l'acide hyaluronique.

Dans un mode de réalisation, ledit polysaccharide est de l'acide hyaluronique ou l'un de ses sels, seul ou en mélange.

Dans un mode de réalisation, ledit polysaccharide est de l'acide hyaluronique sous forme de sel de sodium ou de potassium.

Dans un mode de réalisation, ledit polysaccharide est de l'acide hyaluronique sous forme de sel de sodium.

Dans un mode de réalisation, ledit polysaccharide est de l'acide hyaluronique réticulé ou l'un de ses sels, seul ou en mélange.

Dans un mode de réalisation, ledit polysaccharide est de l'acide hyaluronique réticulé ou l'un de ses sels, seul ou en mélange, et en ce que ladite réticulation est réalisée au moyen d'au moins un agent de réticulation.

Dans un mode de réalisation, ledit polysaccharide est de l'acide hyaluronique réticulé ou l'un de ses sels, seul ou en mélange, et en ce que ladite réticulation est réalisée au moyen d'au moins un agent de réticulation bi- ou polyfonctionnel.

Dans un mode de réalisation, ledit au moins un agent réticulant est choisi dans le groupe constitué de l'éther d'éthylèneglycoldiglycidyle, de l'éther de butanedioldiglycidyle (BDDE), de l'éther de polyglycérolpolyglycidyle, de l'éther de polyéthylèneglycoldiglycidyle, de l'éther de polypropylèneglycoldiglycidyle, d'un bis- ou polyépoxy tel que 1,2,3,4-diépoxybutane ou 1,2,7,8-diépoxyoctane, d'une dialkylsulfone, de la divinylsulfone, du formaldéhyde, de l'épichlorohydrine ou bien encore du glutaraldéhyde, des carbodiimides tels que par exemple le 1-éthyl-3- 3-dimethylaminopropyl carbodiimide hydrochloride (EDC), des trimétaphosphates, comme par exemple le trimétaphosphate de sodium, le trimétaphosphate de calcium, ou encore le trimétaphosphate de barium.

Dans un mode de réalisation, ledit au moins un agent réticulant est choisi dans le groupe constitué de l'éther d'éthylèneglycoldiglycidyle, de l'éther de butanedioldiglycidyle (BDDE), de l'éther de polyglycérolpolyglycidyle, de l'éther de polyéthylèneglycoldiglycidyle, de l'éther de polypropylèneglycoldiglycidyle, d'un bis- ou polyépoxy tel que 1,2,3,4-diépoxybutane ou 1,2,7,8-diépoxyoctane, des trimétaphosphates, comme par exemple le trimétaphosphate de sodium, le trimétaphosphate de calcium, ou encore le trimétaphosphate de barium.

Dans un mode de réalisation, ledit polysaccharide est de l'acide hyaluronique co-réticulé ou l'un de ses sels, seul ou en mélange.

Dans un mode de réalisation, ledit polysaccharide est de l'acide hyaluronique modifié chimiquement par substitution, réticulé ou non réticulé, ou l'un de ses sels, seul ou en mélange.

Dans un mode de réalisation, ledit polysaccharide est de l'acide hyaluronique doublement réticulé tel que décrit dans la demande de brevet WO2000/046253 au nom de Fermentech medical limited.

Dans un mode de réalisation, ledit polysaccharide est un mélange d'acides hyaluroniques, ou l'un de leurs sels, réticulé et non réticulé.

Dans un mode de réalisation, ledit polysaccharide est un mélange d'acides hyaluroniques, ou l'un de leurs sels, réticulés.

Dans un mode de réalisation, ledit polysaccharide est un mélange d'acides hyaluroniques, ou l'un de leurs sels, réticulés tel que celui décrit dans la demande de brevet WO2009/071697 au nom de la demanderesse.

Dans un mode de réalisation, ledit polysaccharide est un mélange d'acides hyaluroniques, obtenu par mélange de plusieurs acides hyaluroniques, ou l'un de leurs sels, de masses moléculaires différentes préalablement à leur réticulation, tel que décrit dans la demande de brevet WO2004092222 au nom de Cornéal industrie.

Dans un mode de réalisation, ledit polysaccharide est de l'acide hyaluronique ou l'un de ses sels, substitué par un groupement apportant des propriétés lipophile ou hydratante, comme par exemple les acides hyaluroniques substitués tels que décrits dans la demande de brevet FR2983483 au nom de la demanderesse. Dans cette demande est décrit un procédé de substitution et réticulation simultanées d'un polysaccharide via ses fonctions hydroxyles, en phase aqueuse, caractérisé en ce qu'il comprend les étapes suivantes : (i) on dispose d'un polysaccharide en milieu aqueux, (ii) on le met en présence d'au moins un précurseur d'un substituant, (iii) on le met en présence d'un agent de réticulation, et (iv) on obtient et on isole le polysaccharide substitué et réticulé.

Dans un mode de réalisation, ledit polysaccharide est de l'acide hyaluronique ou l'un de ses sels, greffé avec du glycérol, par exemple tel que décrit dans la demande WO2017162676 au nom de MERZ.

Dans un mode de réalisation, ledit gel comprend en outre au moins un actif choisi dans le groupe constitué des anesthésiques locaux, des dérives de vitamine C, des antiinflammatoires, des polyols, et de leurs mélanges.

Dans un mode de réalisation, ledit gel comprend en outre au moins un anesthésique local choisi dans le groupe constitué de la lidocaïne, de la mépivacaïne, et de leurs mélanges.

Dans un mode de réalisation, l'au moins un anesthésique local est présent à une concentration en anesthésique local comprise entre 0,1 et 5%, par rapport à la masse totale dudit gel.

Dans un mode de réalisation, ledit gel comprend en outre au moins un antiinflammatoire choisi dans le groupe constitué des antiinflammatoires stéroïdiens et non stéroïdiens.

Dans un mode de réalisation, ledit au moins un antiinflammatoire est choisi dans le groupe constitué des antiinflammatoires stéroïdiens (comme par exemple la dexaméthasone, la prednisolone, la corticostérone, le budésonide, la sulfasalazine, la mésalamine, la cétirizine, la diphénhydramine, l'antipyrine, le salicylate de méthyle, la loratadine, le thymol, le carvacrol, le bisabolol, l'allantoïne, l'eucalyptol, la phénazone (antipyrine), la propyphénazone) et non stéroïdiens (comme par exemple l'ibuprofène, le naproxène, le fénoprofène, le kétoprofène, le flurbiprofène, l'oxaprozine, l'indométacine, le sulindac, l'étodolac, le kétorolac, le diclofénac, la nabumétone, le piroxicam, le méloxicam, le ténoxicam, le droxicam, le lornoxicam, l'isoxicam, l'acide méfénamique, l'acide méclofénamique, l'acide flufénamique, l'acide tolfénamique, le célécoxib, le rofécoxib, le valdécoxib, le parécoxib, le lumiracoxib, l'étoricoxib, le firocoxib, ou encore le sucrose octasulfate et/ou ses sels).

Dans un mode de réalisation, le procédé de préparation d'une formulation comprenant au moins un polymère réticulé obtenu selon le procédé de l'invention comprend en outre au moins une étape d'ajout d'au moins un polyol choisi dans le groupe constitué du mannitol, du sorbitol, du glycérol, du maltitol, du lactitol et de l'érythritol.

Dans un mode de réalisation, ledit gel comprend en outre au moins un polyol choisi dans le groupe constitué du mannitol, du sorbitol et du glycérol.

Dans un mode de réalisation, l'au moins un polyol est présent à une concentration en polyol comprise entre 0,1 mg/ml et 50 mg/ml, par rapport à la masse totale dudit gel.

Dans un mode de réalisation, la masse moléculaire Mw de l'au moins un acide hyaluronique avant réticulation est comprise dans un intervalle de 0,01 MDa et 5 MDa.

Dans un mode de réalisation, la masse moléculaire Mw de l'au moins un acide hyaluronique avant réticulation est comprise dans un intervalle de 0,1 MDa et 3,5 MDa.

Dans un mode de réalisation, la masse moléculaire Mw de l'au moins un acide hyaluronique avant réticulation est comprise dans un intervalle de 1 MDa et 3 MDa.

Dans un mode de réalisation, la masse moléculaire Mw de l'au moins un acide hyaluronique avant réticulation est de 1 MDa.

Dans un mode de réalisation, la masse moléculaire Mw de l'au moins un acide hyaluronique avant réticulation est de 3 MDa.

Dans un mode de réalisation, la concentration en acide hyaluronique HA est comprise entre 2 mg/g et 50 mg/g de poids total dudit l'hydrogel.

Dans un mode de réalisation, la concentration en acide hyaluronique HA est comprise entre 4 mg/g et 40 mg/g de poids total dudit l'hydrogel.

Dans un mode de réalisation, la concentration en acide hyaluronique HA est comprise entre 5 mg/g et 30 mg/g de poids total dudit l'hydrogel.

Dans un mode de réalisation, la concentration en acide hyaluronique HA est comprise entre 10 mg/g et 30 mg/g de poids total dudit l'hydrogel.

Dans un mode de réalisation, la concentration en acide hyaluronique HA est de 20 mg/g de poids total dudit l'hydrogel.

Les applications des gels dont les propriétés rhéologiques sont étudiées en appliquant le procédé selon l'invention sont des applications visant à créer des volumes, à appliquer des contraintes mécaniques et à créer des barrières physiologiques.

Parmi les applications visées on s'intéresse aux applications médicales, on citera par exemple les injections pour remplacer les liquides biologiques déficients, par exemple dans les articulations pour remplacer le liquide synovial, l'injection par suite d'une chirurgie pour éviter les adhérences péritonéales, les injections périurétrales pour traiter l'incontinence et les injections suite à une chirurgie de la presbytie. Parmi les applications esthétiques, on citera par exemple les injections pour le comblement des rides, des ridules et des défauts cutanés ou l'augmentation des volumes par exemple ceux des lèvres, des pommettes, etc.

Les applications visées sont plus particulièrement les applications communément répandues dans le cadre des viscoélastiques injectables et des polysaccharides utilisés ou potentiellement utilisables dans les pathologies ou traitements suivants :
- injections esthétiques au niveau du visage : de comblement de rides, défauts cutanés ou volumatrices (pommettes, menton, lèvres) ;
- injections volumatrices au niveau du corps : augmentation de la taille du mollet, augmentation des seins et des fesses, augmentation du point G, vaginoplastie, reconstruction des lèvres vaginales, augmentation de la taille du pénis ;
- en chirurgie articulaire et en chirurgie dentaire pour le comblement des poches parodontales par exemple.
- traitement de l'arthrose, injection dans l'articulation en remplacement ou en complément du liquide synovial déficient ;
- injection péri-urétrale pour le traitement de l'incontinence urinaire par insuffisance sphinctérienne ;
- injection post-chirurgicale pour éviter les adhérences péritonéales notamment ;
- injection suite à une chirurgie de la presbytie par incisions sclérales au laser ;
- injection dans la cavité vitréenne ;
- injection au cours de la chirurgie de la cataracte ;
- injection pour le traitement des cas de sécheresses vaginales ;
- injection dans les parties génitales.
- pour le comblement des rides fines, moyennes ou profondes, et être injectées avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien à l'emplacement de l'injection.

Ces exemples d'utilisation ne sont nullement limitatifs car les gels étudiés selon le procédé de l'invention ont de nombreuses applications comme par exemple :
- combler des volumes ;
- générer des espaces au sein de certains tissus, favorisant ainsi leur fonctionnement optimal ;
- remplacer des liquides physiologiques déficients.

La présente invention comprend également un procédé de comparaison et de différenciation de gels comprenant les étapes de :
- Détermination, selon le procédé de l'invention ci-dessus décrit, de la plage de résistance de gels, ladite plage de résistance étant définie par la différence ζ_{C} - ζ_{P}; soit l'étendue du domaine plastique en contrainte,
- détermination selon le procédé de l'invention ci-dessus décrit, de la plage de malléabilité de gels, ladite plage de malléabilité étant définie par la différence γ_{C}-γ_{P} ; soit l'étendue du domaine plastique en déformation,
- utilisation des valeurs de plage de résistance et de plage de malléabilité, afin de comparer et différencier les gels.

Dans un mode de réalisation, le procédé de comparaison comprend l'utilisation, en combinaison avec l'utilisation des valeurs de plage de résistance et de plage de malléabilité, des valeurs de module élastique G', de module visqueux G", de delta (δ)et de tangente δ.

Dans un mode de réalisation, le procédé de comparaison des hydrogels permet de comparer une gamme complète d'hydrogels issue d'un même fabricant à partir d'un ou plusieurs paramètres définis ci-dessus.

Dans un mode de réalisation, le procédé de comparaison des hydrogels permet de comparer une gamme de gels de comblement issue d'un même fabricant à partir d'un ou plusieurs paramètres définis ci-dessus.

Dans un mode de réalisation, le procédé de comparaison des hydrogels permet de comparer une gamme de gels volumateurs du visage issue d'un même fabricant à partir d'un ou plusieurs paramètres définis ci-dessus.

Dans un mode de réalisation, le procédé de comparaison des hydrogels permet de comparer une gamme complète d'hydrogels issue de différents fabricants à partir d'un ou plusieurs paramètres définis ci-dessus.

Dans un mode de réalisation, le procédé de comparaison des hydrogels permet de comparer une gamme de gels de comblement issue de différents fabricants à partir d'un ou plusieurs paramètres définis ci-dessus.

Dans un mode de réalisation, le procédé de comparaison des hydrogels permet de comparer une gamme de gels volumateurs du visage issue de différents fabricants à partir d'un ou plusieurs paramètres définis ci-dessus.

### Figures :

La Figure 1 est une représentation schématique de l'évolutions des modules visqueux et élastique d'un échantillon de gel soumis à un balayage en déformation et/ou en contrainte et des domaines de linéarité noté LI, plastique PL et d'étalement ET.
Les Figure 2 et Figure 3, sont des représentations de l'étendue du domaine plastique en contrainte de différentes formules avec en abscisse ζ en Pa et en ordonnées F0.
Les Figure 4 et Figure 5 sont des représentations de l'étendue du domaine plastique en déformation de différentes formules avec en abscisse γ en (%) et en ordonnée F0.
Les Figure 7, Figure 10 et Figure 13 sont des représentations de l'étendue du domaine plastique en contrainte de différentes formules avec en abscisse ζ en Pa et en ordonnées les différents gels étudiés.
Les Figure 8, Figure 11 et Figure 14 sont des représentations de l'étendue du domaine plastique en déformation de différentes formules avec en abscisse γ en (%) et en ordonnée les différents gels étudiés.

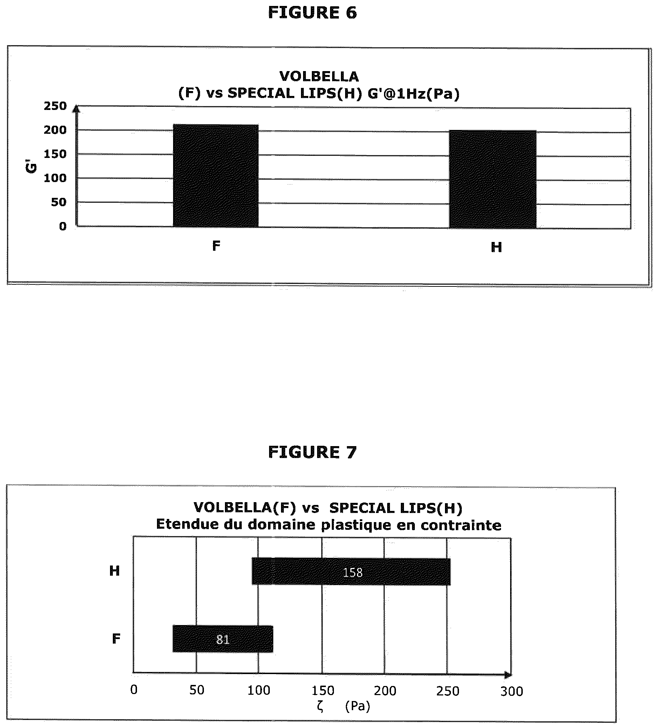

Les Figure 6, Figure 9 et Figure 12 sont des représentations des modules élastiques G' en Pa à 1Hz de différents produits.

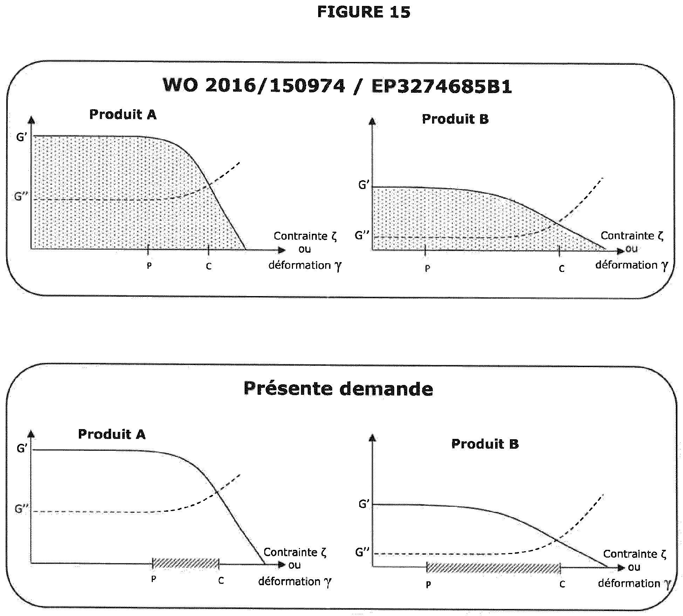

La Figure 15 est une représentation des différences entre la méthode divulguée dans la demande de brevet WO2016/150974 ou dans le brevet EP3274685B1 et la méthode selon la présente invention.

### Exemples :

Les mesures sont effectuées sur le rhéomètre à contrainte imposée DHR-2 (TA Instruments) à l'aide d'une géométrie cône-plan 2° 40mm à 25°C.

Les paramètres de viscoélasticité sont évalués au travers d'un balayage en déformation réalisé de 0,1 à 1000% de déformation à la fréquence de 1Hz. La valeur des modules élastiques et visqueux G' et G" à la fréquence 1Hz correspond à celle des modules élastiques et visqueux dans le domaine linéaire.

Pour l'ensemble des exemples, G'p est la valeur de G' pour laquelle le module élastique a diminué de 10% par rapport à la valeur de G' au plateau du domaine linéaire.

### Exemple 1 :

Le tableau 1 ci-dessous présente les paramètres rhéologiques mesurés, selon le procédé de l'invention, d'un gel d'acide hyaluronique non réticulé.

**Tableau 1**

| Produit | G' @ 1 Hz (Pa) | G" @ 1Hz (Pa) | tanδ @ 1 Hz | γ_{P} (%) | ζ_{P} (Pa) | γ_{C} (%) | ζ_{C} (Pa) | γ_{C}-γ_{P} (%) | ζ_{C}-ζ_{P} (Pa) |
|---|---|---|---|---|---|---|---|---|---|
| A | 1100 | 319 | 0,29 | 36 | 375 | 192 | 813 | 156 | 439 |
| B | 788 | 344 | 0,44 | 40 | 322 | 230 | 863 | 190 | 541 |

Ce gel d'acide hyaluronique non réticulé a été préparé par mise en solution de hyaluronate de sodium de masse moléculaire 3 MDa à la concentration de 30mg/mL dans du tampon PBS.

Le procédé selon l'invention a permis d'évaluer les paramètres rhéologiques du gel avant et après l'étape de stérilisation du gel.

Dans le cadre de cet exemple, le gel avant l'étape de stérilisation est noté A et le gel après l'étape de stérilisation est noté B.

Dans le tableau ci-dessus, on observe une variation des paramètres rhéologiques lors de l'étape de stérilisation du gel d'acide hyaluronique.

On note une augmentation de la plage de malléabilité et une diminution de la plage de résistance au cours de cette étape de stérilisation.

### Exemple 2 :

Le tableau ci-dessous présente les paramètres rhéologiques mesurés, selon le procédé de l'invention, d'un gel KartilageCross^{®} (injection intra-articulaire) commercialisé par les Laboratoires Vivacy.

**Tableau 2**

| Produit | G' @ 1Hz (Pa) | G" @ 1Hz (Pa) | tanδ @ 1 Hz | γ_{P} (%) | ζ_{P} (Pa) | γ_{C} (%) | ζ_{C} (Pa) | γ_{C}-γ_{P} (%) | ζ_{C}-ζ_{P}(Pa) |
|---|---|---|---|---|---|---|---|---|---|
| C | 169,3 | 26,9 | 0,159 | 40 | 63 | 253 | 182 | 213 | 119 |

Dans le cadre de cet exemple, le gel KartilageCross commercialisé par les Laboratoires VIVACY est noté C.

Le procédé selon l'invention a permis de mesurer et d'évaluer les paramètres rhéologiques classiques du gel C comme le module élastique et le module visqueux.

En outre, le procédé selon l'invention a permis de mesurer des paramètres rhéologiques comme la plage de malléabilité et la plage de résistance du gel C.

Ces paramètres permettent notamment de compléter l'étude du comportement du gel C.

### Exemple 3 :

Le tableau 3 ci-dessous présente les paramètres rhéologiques mesurés, selon le procédé de l'invention, de deux formulations de gels.

**Tableau 3**

| Stérilisation vapeur | Produit | G' @1Hz (Pa) | G" @1Hz (Pa) | YP (%) | ζ_{P} (Pa) | γ_{C} (%) | ζ_{C} (Pa) | γ_{C}-γ_{P} (%) | ζ_{C}-ζ_{P} (Pa) |
|---|---|---|---|---|---|---|---|---|---|
| Non | D | 367 | 43 | 54 | 183 | 417 | 540 | 363 | 358 |
| | E | 379 | 39 | 51 | 176 | 384 | 511 | 333 | 334 |
| F₀= 9min | D | 311 | 44 | 57 | 164 | 494 | 542 | 436 | 378 |
| | E | 284 | 40 | 64 | 167 | 508 | 529 | 444 | 362 |
| F₀=20,5min | D | 282 | 42 | 64 | 167 | 538 | 544 | 474 | 377 |
| | E | 238 | 37 | 64 | 140 | 595 | 527 | 531 | 387 |
| F₀=46,5min | D | 242 | 41 | 64 | 143 | 619 | 559 | 555 | 415 |
| | E | 171 | 31 | 64 | 101 | 766 | 560 | 703 | 458 |

La première est une formulation réalisée selon le procédé décrit dans le brevet EP231108 avec incorporation de mannitol (notée D dans le tableau ci-dessus).

La seconde est une formulation réalisée selon le procédé dans le brevet EP231108 sans incorporation de mannitol (notée E dans le tableau ci-dessus).

Le procédé selon l'invention a permis d'évaluer les paramètres rhéologiques de ces deux formulations à différentes valeurs stérilisatrices F₀= 9min ; F₀=20,5min et F₀=46,5min.

Pour rappel, la valeur stérilisatrice est exprimée en unité de temps et permet de quantifier l'effet d'un traitement stérilisant.

Dans le tableau ci-dessus, on observe que le module élastique des formulations D et E diminue au cours de l'étape de stérilisation.

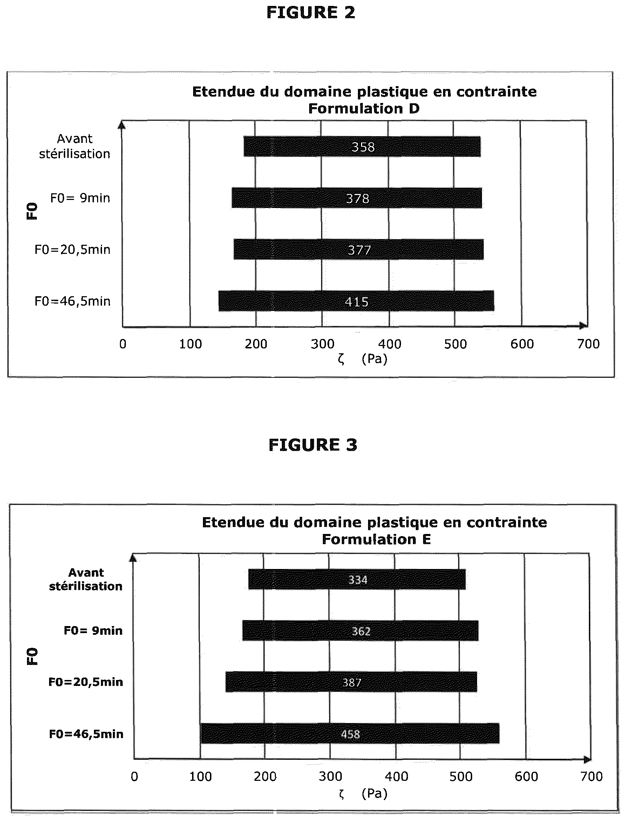

En revanche, comme illustré sur les Figure 2 et Figure 3, le paramètre de plage de résistance augmente lors de la stérilisation des formulations D et E.

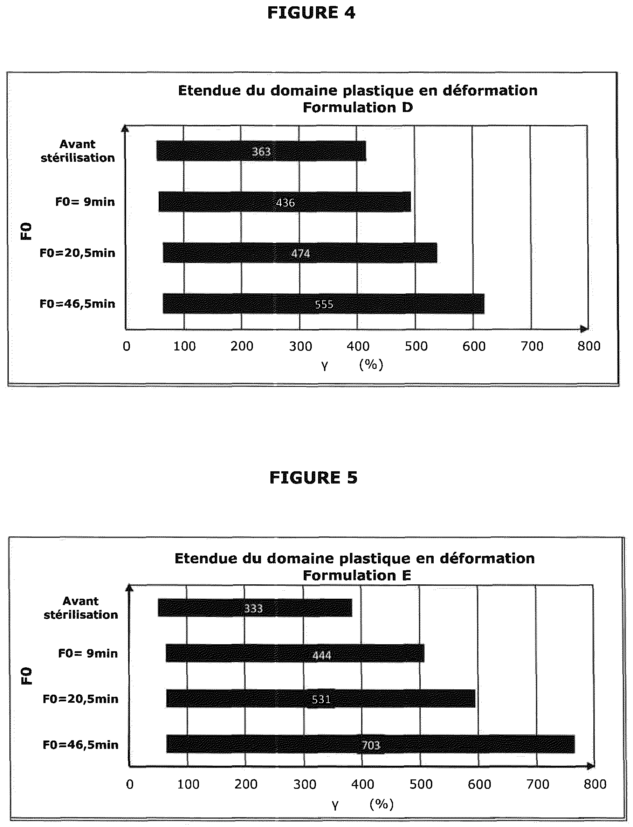

Comme illustré sur les Figure 4 et Figure 5, l'étendue de la plage de malléabilité augmente significativement lors du procédé de stérilisation des formulations D et E.

### Exemple 4 :

Le tableau ci-dessous présente les paramètres rhéologiques mesurés, selon le procédé de l'invention, de deux gels différents.

**Tableau 4**

| Produit | G' @ 1Hz (Pa) | G" @ 1Hz (Pa) | tanδ @ 1Hz | γ_{P} (%) | ζ_{P} (Pa) | γ_{C} (%) | ζ_{C} (Pa) | γ_{C}-γ_{P} (%) | ζ_{C}-ζ_{P}(Pa) |
|---|---|---|---|---|---|---|---|---|---|
| F | 213 | 25 | 0,12 | 16 | 31 | 128 | 112 | 112 | 81 |
| H | 204 | 33 | 0,16 | 51 | 95 | 309 | 253 | 258 | 158 |

Le premier est le produit Juvederm Volbella (avec lidocaïne) commercialisé par la société Allergan. Dans le cadre de cet exemple, le produit Volbella est noté F.

La seconde est le produit Special Lips de la gamme Stylage commercialisé par les Laboratoires Vivacy. Dans le cadre de cet exemple, le produit Special Lips est noté H.

Comme illustré à la Figure 6, les gels F et H présentent des modules élastiques G' similaires.

Ils présentent également des modules visqueux G" voisins.

En revanche, l'étendue de la plage de résistance et de la plage de malléabilité permettent de distinguer les deux produits.

Comme illustré à la Figure 7, la plage de résistance du produit H est deux fois plus étendue que celle du produit F, elle est également déplacée en translation vers des valeurs plus élevées de ζ de telle façon qu'il n'y a pratiquement pas de recouvrement des plages de résistance.

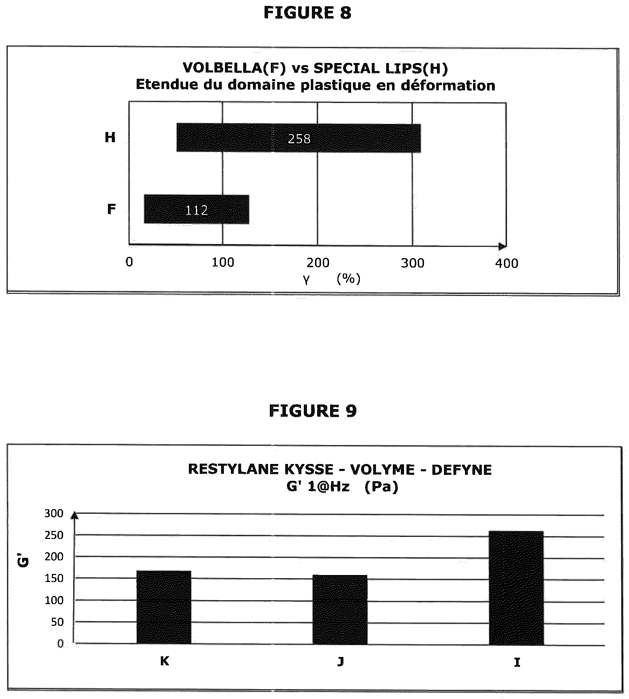

Également à la Figure 8, on observe que la plage de malléabilité du produit H est deux fois plus étendue que celle du produit F, elle est également déplacée en translation vers des valeurs plus élevées de ζ mais un recouvrement important des plages de résistance est conservé.

En conclusion, le procédé selon l'invention a permis de différencier deux gels avec des modules élastiques et visqueux similaires.

### Exemple 5 : Le tableau ci-dessous présente les paramètres rhéologiques mesurés, selon le procédé de l'invention, de trois gels Restylane différents

**Tableau 5**

| Produit | G' @ 1Hz (Pa) | G" @ 1Hz (Pa) | Tanδ @ 1Hz | γ_{P} (%) | ζ_{P} (Pa) | γ_{C} (%) | ζ_{C} (Pa) | ζ_{C}-ζ_{P} (Pa) | γ_{C}-γ_{P} (%) |
|---|---|---|---|---|---|---|---|---|---|
| 1 | 262,5 | 26,01 | 0,099 | 32 | 77 | 631 | 664 | 587 | 599 |
| J | 160 | 25 | 0,16 | 64 | 94 | 942 | 679 | 585 | 878 |
| K | 168 | 25 | 0,15 | 64 | 98 | 874 | 660 | 562 | 810 |

Le premier est le produit Restylane Defyne commercialisé par la société Galderma. Dans le cadre de cet exemple, le produit Restylane Defyne est noté I.

Le second est le produit Restylane Volyme commercialisé par la société Galderma. Dans le cadre de cet exemple, le produit Restylane Volyme est noté J.

Le troisième est le produit Restylane Kysse commercialisé par la société Galderma. Dans le cadre de cet exemple, le produit Restylane Kysse est noté K.

Comme illustré à la Figure 9, les gels J et K présentent des modules élastiques G' similaires tandis que le module élastique du gel I est différent.

En revanche, les gels I, J et K présentent des modules visqueux G" similaires.

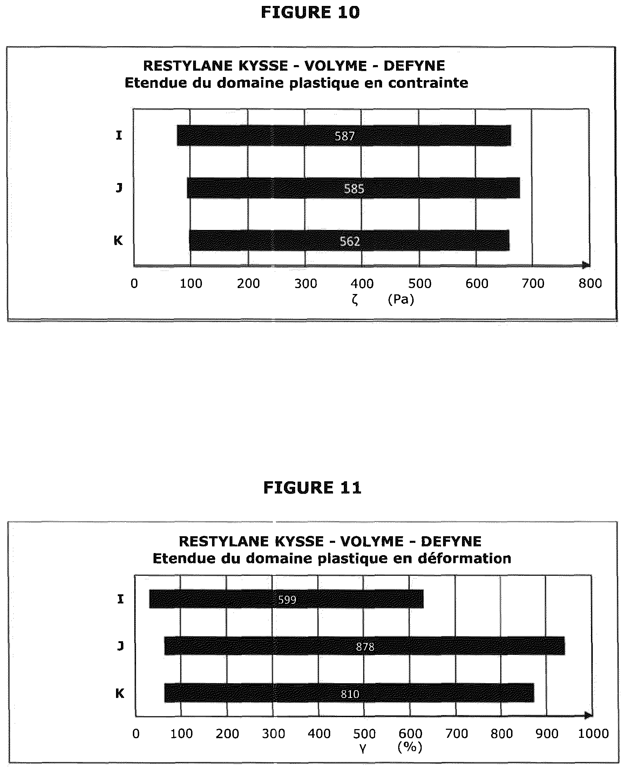

Comme indiqué sur la Figure 10, la plage de résistance des gels I et J est quasi-identique et est du même ordre de grandeur pour le gel K.

Comme indiqué sur la Figure 11, on observe que la plage de malléabilité diffère selon la nature des produits I, J et K.

Dans le cadre de cet exemple, le procédé selon l'invention a permis de différencier deux gels avec des modules élastiques et visqueux similaires (J et K).

En conclusion, le procédé selon l'invention a permis d'évaluer les paramètres rhéologiques de gels commerciaux et de différencier deux gels avec des modules élastiques et visqueux similaires.

### Exemple 6 :

Le tableau ci-dessous présente les paramètres rhéologiques mesurés, selon le procédé de l'invention, de deux gels différents.

**Tableau 6**

| | G' @ 1Hz (Pa) | G"@ 1Hz (Pa) | γ_{P} (%) | ζ_{P} (Pa) | γ_{C} (%) | ζ_{C} (Pa) | γ_{C}-γ_{P} (%) | ζ_{C}-ζ_{P} (Pa) |
|---|---|---|---|---|---|---|---|---|
| L | 50 | 4 | 101 | 46 | 346 | 90 | 245 | 44 |
| M | 71 | 5 | 90 | 58 | 442 | 158 | 352 | 100 |

La solution de gélatine bovine à 2% non stérile, notée L, a été obtenue à partir de gélatine type « high bloom » après dissolution dans de l'eau purifiée, homogénéisation par vortex puis chauffée à 37°C pendant 15 minutes, suivi d'une nouvelle homogénéisation et enfin refroidissement à température ambiante.

La solution de gélatine porcine à 2% non stérile, notée M, a été obtenue à partir de gélatine type « high bloom » après dissolution dans de l'eau purifiée, homogénéisation par vortex puis chauffée à 37°C pendant 15 minutes, suivi d'une nouvelle homogénéisation et enfin refroidissement à température ambiante.

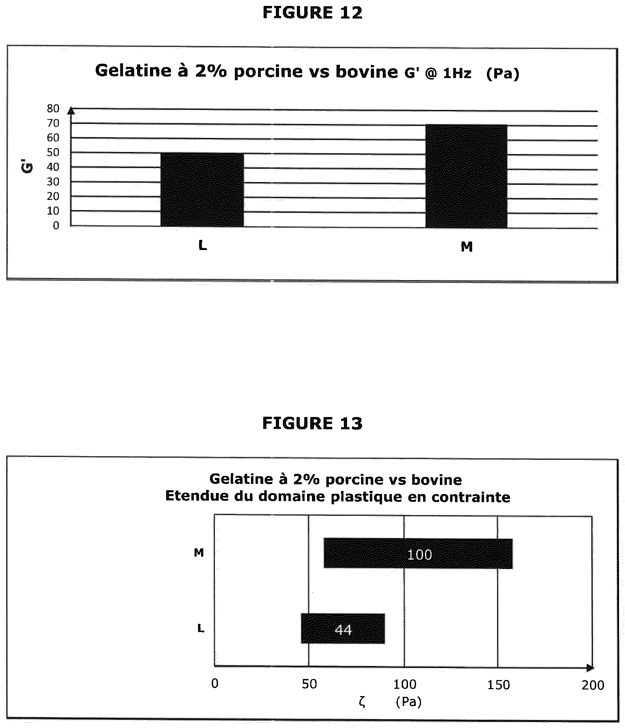

Comme illustré à la Figure 12, les gels L et M présentent des modules élastiques G' voisins.

Ils présentent également des modules visqueux G" similaires.

Comme illustre à la Figure 13, la plage de résistance du produit M est deux fois plus étendue que celle du produit L et elle est également déplacée en translation vers des valeurs plus élevées de ζ mais un recouvrement important des plages de résistance est conservée.

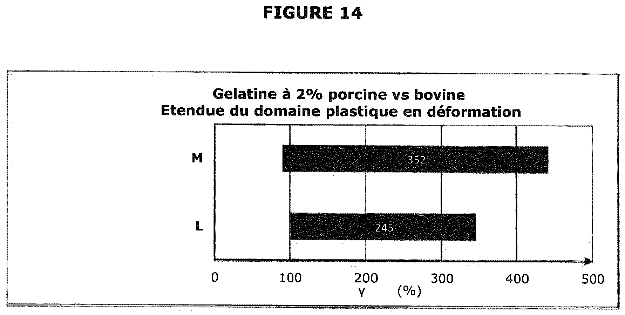

Également à la Figure 14, on observe que la plage de malléabilité du produit M est plus étendue que celle du produit L.

Ces paramètres permettent notamment de compléter l'étude du comportement des gels L et M.

En conclusion, le procédé selon l'invention a permis d'évaluer et de comparer les paramètres rhéologiques des gels de gélatine comme les gels L et M.

## Revendications

1. Procédé d'évaluation des caractéristiques rhéologiques d'au moins un gel, consistant en la détermination de l'étendue du domaine plastique en contrainte ζ_{C} -ζ_{P} et en déformation γ_{C}-γ_{P}, ladite détermination étant réalisée selon les étapes de :
- soumission d'au moins un échantillon d'au moins un gel à des contraintes mécaniques oscillatoires à une fréquence fixée,
- détermination et tracé des courbes du module élastique G' et du module visqueux G" en fonction de la déformation et de la contrainte,
- détermination de ζ_{C} et γ_{C} au point d'intersection X_{c} des courbes G' et G" en contrainte et en déformation,
- détermination de ζ_{P} et γ_{P} par fixation d'une valeur arbitraire de G' (G'x) que l'on définira comme la valeur d'entrée dans le domaine plastique et,
- calcul de ζ_{C} -ζ_{P} et de γ_{C}-γ_{P}.

2. Procédé selon la revendication 1, **caractérisé en ce que** les contraintes mécaniques oscillatoires sont mises en oeuvre par un balayage en amplitude.

3. Procédé selon la revendication 2, **caractérisé en ce que** le balayage en amplitude est un balayage en déformation et/ou en contrainte.

4. Procédé selon la revendication 1 et 2, **caractérisé en ce que** la valeur arbitraire de G' est définie comme la valeur pour laquelle le module élastique G' a diminué d'au plus 15% par rapport à la valeur de G' au plateau du domaine linéaire.

5. Procédé selon l'une quelconque des revendications précédentes, **caractérisé en ce que** la fréquence fixée est comprise entre 0,1 et 10 Hz.

6. Procédé selon l'une quelconque des revendications précédentes, **caractérisé en ce que** la fréquence fixée est égale à 1 Hz.

7. Procédé selon l'une quelconque des revendications précédentes, **caractérisé en ce qu'**il est mis en oeuvre par l'utilisation d'un rhéomètre relié à une unité de contrôle pour effectuer les mesures et les calculs.

8. Procédé selon l'une quelconque des revendications précédentes, **caractérisé en ce que** le gel est un hydrogel.

9. Procédé selon l'une quelconque des revendications précédentes, **caractérisé en ce que** l'hydrogel est constitué d'au moins un polysaccharide choisi dans le groupe comprenant : de l'acide hyaluronique, de l'héparosan, du kératane, de l'héparine, de la cellulose, des dérivés de cellulose (notamment l'hydroxypropylcellulose, l'hydroxypropylméthylcellulose, l'éthylméthyle cellulose, la carboxyméthylcellulose) de l'acide alginique, du xanthane, du carraghénane, du chitosan, de la chondroitine, l'héparosan, et leurs sels biologiquement acceptables, seul(s) ou en mélange.

10. Procédé selon l'une quelconque des revendications précédentes, **caractérisé en ce que** le gel est constitué d'acide hyaluronique.

11. Procédé selon l'une quelconque des revendications précédentes, **caractérisé en ce que** le gel est un gel injectable.

12. Procédé de comparaison et de différenciation de gels comprenant les étapes de :
- détermination selon le procédé de la revendication 1 de la plage de résistance de gels, ladite plage de résistance étant définie par la différence ζ_{C}-ζ_{P}; soit l'étendue du domaine plastique en contrainte,
- détermination selon le procédé de la revendication 1 de la maléabilité de gels, ladite plage de malléabilité étant définie par la différence γ_{C}-γ_{P} ; soit l'étendue du domaine plastique en déformation,
- utilisation des paramètres définis ci-dessus afin de comparer et différencier les gels.

## Patentansprüche

1. Verfahren zur Bewertung der rheologischen Eigenschaften mindestens eines Gels, umfassend Bestimmen des Ausmaßes des plastischen Bereichs unter Spannung ζc-ζp und unter Verformung γ_{C}-γ_{P}, wobei das Bestimmen gemäß den folgenden Schritten durchgeführt wird:
- Beaufschlagen mindestens einer Probe von mindestens einem Gel mit oszillierenden mechanischen Spannungen bei einer festen Frequenz,
- Bestimmen und Darstellen der Kurven des Elastizitätsmoduls G' und des Viskositätsmoduls G" als eine Funktion der Verformung und der Spannung,
- Bestimmen von ζ_{C} und γ_{C} am Schnittpunkt Xc der Kurven von G' und G" unter Spannung und unter Verformung,
- Bestimmen von ζp und γp durch Festlegen eines beliebigen Wertes von G' (G'x), der als Eintrittswert in den plastischen Bereich definiert ist, und
- Berechnen von ζc-ζp und γc-γp.

2. Verfahren nach Anspruch 1, **dadurch gekennzeichnet, dass** die oszillierenden mechanischen Spannungen mittels eines Amplituden-Durchlaufs implementiert werden.

3. Verfahren nach Anspruch 2, **dadurch gekennzeichnet, dass** der Amplituden-Durchlauf ein Verformungs- und/oder Spannungs-Durchlauf ist.

4. Verfahren nach Anspruch 1 und 2, **dadurch gekennzeichnet, dass** der beliebige Wert von G' als der Wert definiert ist, bei dem der Elastizitätsmodul G' um höchstens 15 % gegenüber dem Wert von G' am Plateau des linearen Bereichs abgenommen hat.

5. Verfahren nach einem der vorangehenden Ansprüche, **dadurch gekennzeichnet, dass** die feste Frequenz zwischen 0,1 und 10 Hz beträgt.

6. Verfahren nach einem der vorangehenden Ansprüche, **dadurch gekennzeichnet, dass** die feste Frequenz 1 Hz beträgt.

7. Verfahren nach einem der vorangehenden Ansprüche, **dadurch gekennzeichnet, dass** es unter Verwendung eines Rheometers durchgeführt wird, das mit einer Steuereinheit verbunden ist, um die Messungen und die Berechnungen durchzuführen.

8. Verfahren nach einem der vorangehenden Ansprüche, **dadurch gekennzeichnet, dass** das Gel ein Hydrogel ist.

9. Verfahren nach einem der vorangehenden Ansprüche, **dadurch gekennzeichnet, dass** das Hydrogel aus mindestens einem Polysaccharid besteht, das aus der Gruppe ausgewählt ist, die aus Hyaluronsäure, Heparosan, Keratan, Heparin, Cellulose, Cellulosederivaten (insbesondere Hydroxypropylcellulose, Hydroxypropylmethylcellulose, Ethylmethylcellulose, Carboxymethylcellulose), Alginsäure, Xanthan, Carrageen, Chitosan, Chondroitin, Heparosan und biologisch verträglichen Salzen davon, allein oder als Mischung, besteht.

10. Verfahren nach einem der vorangehenden Ansprüche, **dadurch gekennzeichnet, dass** das Gel aus Hyaluronsäure besteht.

11. Verfahren nach einem der vorangehenden Ansprüche, **dadurch gekennzeichnet, dass** das Gel ein injizierbares Gel ist.

12. Verfahren zum Vergleichen und Unterscheiden von Gelen, umfassend die folgenden Schritte:
- Bestimmen des Widerstandsbereichs von Gelen gemäß dem Verfahren nach Anspruch 1, wobei der Widerstandsbereich durch die Differenz ζc-ζp definiert ist, die das Ausmaß des plastischen Bereichs unter Spannung darstellt,
- Bestimmen der Formbarkeit von Gelen gemäß dem Verfahren nach Anspruch 1, wobei der Formbarkeitsbereich durch die Differenz γc-γp definiert ist, die das Ausmaß des plastischen Bereichs unter Verformung darstellt,
- Verwenden der oben definierten Parameter zum Vergleichen und Unterscheiden der Gele.

## Claims

1. A method for evaluating the rheological properties of at least one gel, comprising determining the extent of the plastic domain in stress ζ_{c}-ζₚ and in strain γ_{c}-γₚ, the determining being carried out according to the steps of:
- subjecting at least one sample of at least one gel to oscillatory mechanical stresses at a fixed frequency,
- determining and plotting the curves of the elastic modulus G' and of the viscous modulus G" as a function of the strain and of the stress,
- determining ζ_{c} and γ_{c} at the point of intersection X_{c} of the curves of G' and G" under stress and strain,
- determining ζₚ and γₚ by fixing an arbitrary value of G' (G'x) that is defined as the entry value into the plastic domain, and
- calculating ζ_{c}-ζₚ and γ_{c}-γₚ.

2. The method as set forth in claim 1, **characterized in that** the oscillatory mechanical stresses are implemented by means of an amplitude sweep.

3. The method as set forth in claim 2, **characterized in that** the amplitude sweep is a strain and/or stress sweep.

4. The method as set forth in claim 1 and 2, **characterized in that** the arbitrary value of G' is defined as the value for which the elastic modulus G' has decreased by at most 15% compared to the value of G' at the plateau of the linear domain.

5. The method as set forth in any one of the preceding claims, **characterized in that** the fixed frequency is between 0.1 and 10 Hz.

6. The method as set forth in any one of the preceding claims, **characterized in that** the fixed frequency is equal to 1 Hz.

7. The method as set forth in any one of the preceding claims, **characterized in that** it is implemented through the use of a rheometer connected to a control unit for carrying out the measurements and the calculations.

8. The method as set forth in any one of the preceding claims, **characterized in that** the gel is a hydrogel.

9. The method as set forth in any one of the preceding claims, **characterized in that** the hydrogel consists of at least one polysaccharide selected from the group consisting of hyaluronic acid, heparosan, keratan, heparin, cellulose, cellulose derivatives (particularly hydroxypropyl cellulose, hydroxypropylmethyl cellulose, ethylmethyl cellulose, carboxymethyl cellulose), alginic acid, xanthan, carrageenan, chitosan, chondroitin, heparosan, and biologically acceptable salts thereof, alone or as a mixture.

10. The method as set forth in any one of the preceding claims, **characterized in that** the gel consists of hyaluronic acid.

11. The method as set forth in any one of the preceding claims, **characterized in that** the gel is an injectable gel.

12. A method for comparing and differentiating gels, comprising the steps of:
- determining, according to the method of claim 1, the resistance range of gels, said resistance range being defined by the difference ζ_{c}-ζₚ, which is the extent of the plastic domain in stress,
- determining, according to the method of claim 1, the malleability of gels, said malleability range being defined by the difference γ_{c}-γₚ, which is the extent of the plastic domain in strain,
- using the parameters defined above in order to compare and differentiate the gels.
